# EUROPÄISCHE PATENTSCHRIFT

(11) **EP 2 097 002 B1**
(45) Veröffentlichungstag und Bekanntmachung des Hinweises auf die Patenterteilung: **17.08.2011**
(21) Anmeldenummer: 07819742.3
(22) Anmeldetag: 10.11.2007
(51) Int. Cl.: A61B 5/103, G06K 9/00

(54) **VORRICHTUNG UND VERFAHREN ZUR AUFNAHME EINES TRITTPROFILS**
DEVICE AND METHOD FOR CAPTURING A GAIT PATTERN
DISPOSITIF ET PROCEDE POUR L'ACQUISITION D'UN PROFIL DE FOULEE

(30) Priorität: 10.11.2006 DE 102006053348; 10.11.2006 DE 202006017301 U
(43) Veröffentlichungstag der Anmeldung: 09.09.2009
(73) Patentinhaber: MLS Lanny GmbH, 75323 Bad Wildbad (DE)
(72) Erfinder: LANNY, Michael, 75323 Bad Wildbad (DE)
(74) Vertreter: Leitner, Waldemar
(86) Internationale Anmeldenummer: PCT/EP2007/009750
(87) Internationale Veröffentlichungsnummer: WO 2008/055699

(56) Entgegenhaltungen:
- DE-C1- 4 027 317
- US-A- 5 029 483
- US-A- 5 186 062
- US-A- 5 626 539
- US-A- 6 010 465

## Beschreibung

Die Erfindung betrifft eine Vorrichtung zur Aufnahme eines Trittprofils eines Lebewesens, insbesondere eines Huftiers, hierbei insbesondere eines Pferdes, die eine Trittplatte und mindestens einen der Trittplatte zugeordneten oder in ihr integrierten Sensor aufweist, wobei ein Sensorsignal des mindestens einen Sensors einer Auswerteeinrichtung zuführbar ist, wobei die Vorrichtung ein Laufband aufweist, das über der Trittplatte angeordnet ist, und wobei durch den mindestens einen Sensor die einzelnen Trittimpulse einer aus einer Abfolge von Einzelbewegungen bestehenden dynamischen Bewegungssequenz erfassbar und die zu den einzelnen Trittimpulsen korrelierten Sensorsignale der Auswerteeinrichtung zuführbar sind, sowie ein Verfahren zur Aufnahme eines Trittprofils eines Lebewesens, insbesondere eines Huftiers, hierbei insbesondere eines Pferdes, bei dem eine Beaufschlagung einer Trittplatte von mindestens einem Sensor erfasst wird und das aus der Beaufschlagung der Trittplatte resultierende Sensorsignal einer Auswerteeinrichtung zugeführt wird, wobei zur Erfassung einzelner Trittimpulse einer aus einer Abfolge von Trittimpulsen bestehenden dynamischen Bewegungssequenz das Lebewesen auf einem über der Tragplatte angeordneten Laufband angeordnet wird, und wobei durch den mindestens einen Sensor die einzelnen Trittimpulse einer aus einer Abfolge von Einzelbewegungen bestehenden dynamischen Bewegungssequenz erfasst und die zu den einzelnen Trittimpulsen korrelierten Sensorsignale der Auswerteeinrichtung zu geführt werden.

Derartige Vorrichtungen und Verfahren sind beispielsweise aus der Humanmedizin und der Orthopädie bekannt und werden zur Erfassung der Belastungssituation von Füßen von Patienten verwendet. Hierzu wird der Patient auf die als Kraftmessvorrichtung ausgeführte Trittplatte gestellt und die derart bewirkte Kraftbeaufschlagung der Trittplatte wird von einer Vielzahl von als Flächenelementen ausgeführten Sensoren erfasst, um die statische Belastung der Trittplatte durch die Füße des Patienten zu ermitteln. Hierdurch wird eine ortsaufgelöste Belastungsverteilung des auf der Trittplatte stehenden Fußes des Patienten erfasst und die Sensorsignale werden zu der Auswerteeinrichtung geführt, auf der das derartig gewonnene Belastungsprofil des Fußes dargestellt und/oder ausgewertet wird, um Aussagen über Belastungsschwerpunkte dieses auf der Trittplatte stehenden Fußes zu gewinnen.

Die bekannten Vorrichtungen und Verfahren weisen jedoch den Nachteil auf, dass mit ihnen nur eine statische Trittanalyse, bei der der Benutzer auf der Trittplatte der Vorrichtung steht, durchgeführt werden kann. Es ist mit den bekannten Vorrichtungen und Verfahren in nachteiliger Art und Weise nicht möglich, einen dynamischen Bewegungsablauf, also eine aus mehreren Trittfolgen bestehende Sequenz, zu analysieren.

Die Notwendigkeit, eine derartige dynamische Bewegungssequenz zu analysieren, tritt nicht nur im Bereich der Humanmedizin, insbesondere der Orthopädie oder der Sportmedizin auf. Vielmehr ist es auch bei Tieren; insbesondere bei Pferden wünschenswert, ein Verfahren und eine Vorrichtung verfügbar zu haben, mit denen eine Analyse eines dynamischen Bewegungsablaufs durch Erfassung des Trittprofils ermöglicht wird. Insbesondere Lahmheitsuntersuchungen bei Pferden waren bisher nur visuell möglich: Derartige Lahmheitsuntersuchungen konnten bis jetzt nur derart ausgeführt werden, dass das Pferd im Schritt dem Tierarzt vorgeführt wurde und er auf den dabei gewonnen optischen Eindruck angewiesen war.

Eine Vorrichtung und ein Verfahren der eingangs genannten Art sind aus der WO 93/06779 bekannt. Diese Druckschrift beschreibt eine Vorrichtung, die eine Trittplatte mit einer Vielzahl ihr zugeordneter Sensoren aufweist, wobei Sensorsignale der Sensoren einer Auswerteinrichtung zuführbar sind. Über der Trittplatte ist ein Laufband vorgesehen. Als Sensoren werden hierbei Kraftsensoren verwendet, die sich unter dem Laufband befinden. Eine derartige Vorrichtung besitzt den Nachteil, dass die Trittplatte in der Vorrichtung starr angeordnet ist. Insbesondere bei Lahmheitsuntersuchungen von Huftieren wie Pferden ist dies von großem Nachteil, da die starre Ausbildung und Anordnung der Trittplatte bewirkt, dass ein "unnatürlicher" Untergrund gegeben ist, welcher die Gangart des Tiers beeinflussen und somit zu einer Verfälschung des Untersuchungsergebnisses führen kann.

Die US-A-51856062 offenbart, dass in einer Führungsbahn für ein Pferd eine ausgedehnte Messfläche ausgebildet wird, die z. B. 4 Meter lang und 0,8 Meter breit und unter einer Abdeckplane angeordnet ist. Die Messfläche weist eine Vielzahl von parallel angeordneten Messsektionen auf, von denen jede kürzer als die Länge eines Pferdehufes und breiter als die Breite eines Pferdes ist. Jede Messsektion weist eine Kraftmesseinrichtung auf, welche eine im wesentlichen rechteckige Kraftmessfläche besitzt. Die Kraftmesseinrichtungen sind in einem Hilfsrahmen angeordnet und jede Kraftmesseinrichtung besitzt einen steifen Querbalken, welche in bezug auf den Hilfsrahmen an beiden Seiten von Messblöcken getragen wird. Die Belastung der Querbalken wird gemessen und die entsprechenden Signale werden ausgewertet. Eine derartige Vorrichtung besitzt den Nachteil, dass hierbei die Messfläche sehr lang ausgeführt werden muss, da das Pferd zur Erfassung eines dynamischen Bewegungsprofils über die Messfläche geführt werden muss. Da die gesamte Messfläche durch die Messsektionen abgedeckt werden muss, ist eine Vielzahl von Kraftmesseinrichtungen erforderlich, deren Signale ausgewertet werden müssen. Besonders deutlich wird dieser Nachteil, wenn man die in der vorgenannten Druckschrift angegebenen exemplarische Ausgestaltung betrachtet: Bei einer empfohlenen Länge des Messfelds von 4 m und einer Länge der einzelnen Messsektionen von 25 mm sind dann 160 Messsektionen und somit 160 Kraftmesseinrichtungen erforderlich, wobei dann die Auswerteeinrichtung der Vorrichtung in der Lage sein muss, 160 Messsignale parallel auszuwerten. Ein derartiger apparativer Aufwand ist in wirtschaftlicher Hinsicht nicht tragbar.

Es ist Aufgabe der vorliegenden Erfindung, eine Vorrichtung und ein Verfahren der eingangs genannten Art derart weiterzubilden, dass in einfacher Art und Weise eine verbesserte Erfassung des Trittprofils einer dynamischen Bewegungssequenz ermöglicht wird.

Zur Lösung dieser Aufgabe schlägt die erfindungsgemäße Vorrichtung vor, dass die Trittplatte mittels einer Lagerung elastisch beweglich in der Vorrichtung gelagert ist, und dass der oder mindestens einer der Sensoren als ein eine Beschleunigung und/oder eine Auslenkung der Trittplatte erfassender Sensor ausgebildet ist.

Das erfindungsgemäße Verfahren sieht vor, dass die Trittplatte elastisch in der Vorrichtung gelagert wird, und dass als der Sensor oder als mindestens einer der Sensoren ein Beschleunigungssensor oder ein Wegmesssensor verwendet wird, wodurch die durch die Beaufschlagung der Trittplatte durch die Extremitäten des Benutzers hervorgerufene Beschleunigung und/oder Auslenkung derselben erfasst wird.

Durch diese erfindungsgemäßen Maßnahmen wird in vorteilhafter Art und Weise ein Verfahren und eine Vorrichtung geschaffen, welche sich durch eine verbesserte Erfassung von Trittimpulse einer dynamischen Bewegungssequenz auszeichnen. Die elastische Lagerung der Trittplatte und somit des auf ihr angeordneten Laufbandes bewirkt, dass die Trittplatte bei einer Beaufschlagung durch eine Extremität des Benutzers nachgibt. Hierdurch kann in vorteilhafter Art und Weise ein natürlicher Untergrund simuliert werden, der zu einer natürlicheren Bewegungsart, insbesondere bei Pferden oder ähnlichen Tieren führt. Die Verwendung eines Beschleunigungssensors oder eines Wegsensors erlaubt es dann, die von den Extremitäten des Benutzers auf die Trittplatte ausgeübten Beschleunigungen bzw. Auslenkungen derselben zu erfassen. Bei der erfindungsgemäßen Vorrichtung wird also in vorteilhafter Art und Weise nicht die auf die Trittplatte einwirkende Kraft erfasst, sondern die Beschleunigung bzw. Auslenkung der Trittplatte selbst, welche durch die Beaufschlagung derselben durch die sich bewegenden Extremitäten des Benutzers hervorgerufen wird. Eine derartige Vorgangsweise besitzt den Vorteil einer verbesserten Genauigkeit und eines einfacheren Aufbaus, als dies bei der Verwendung einer Vielzahl von Kraftsensoren der Fall wäre, welche außerdem ständig vom Gewicht des auf der Trittplatte stehenden Benutzers beaufschlagt würden. Es ist des weiteren bei der erfindungsgemäßen Vorrichtung nicht mehr erforderlich, dass eine Vielzahl von Sensoren vorgesehen ist, da prinzipiell bereits mit einem einzigen Sensor die Beschleunigung bzw. die Auslenkung der Trittplatte erfassbar ist. Die erfindungsgemäßen Maßnahmen erlauben es also, nicht nur eine Analyse einer statischen Belastungssituation vorzunehmen, sondern in vorteilhafter Art und Weise eine gleichzeitige Abfolge von Bewegungsschritten, also eine dynamische Bewegungssequenz, durch die Erfassung der entsprechenden Trittimpulse in einfacher Art und Weise zu analysieren.

Eine weitere vorteilhafte Weiterbildung der Erfindung sieht vor, dass die Steifigkeit der Lagerung der Trittplatte variabel einstellbar ist. Hierdurch ist es beispielsweise möglich, die Auslenkung der Trittplatte auf individuelle Anforderungen des Benutzers anzupassen, indem zum Beispiel bei Benutzern mit hohem Gewicht eine hohe Steifigkeit und bei Benutzern mit geringem Gewicht eine geringere Steifigkeit gewählt wird. Weiterhin können dadurch unterschiedliche Laufuntergründe simuliert werden, wobei eine hohe Steifigkeit einen harten Untergrund und eine geringe Steifigkeit einen weichen Untergrund nachbildet.

Eine vorteilhafte Weiterbildung der Erfindung sieht vor, dass die zu den einzelnen Trittimpulsen der dynamischen Bewegungssequenz korrelierten Sensorsignale auf einer Anzeigeeinheit der Auswerteeinrichtung darstellbar sind, wobei vorzugsweise die Auswerteeinrichtung bei der Abweichung eines Trittimpulses von einem anderen Trittimpuls und/oder von einem vorgegeben Norm-Trittimpuls ein Signal erzeugt. Auf diese Art und Weise ist in besonders einfacher Art und Weise eine Diagnose von Fehlbelastungen auch von ungeübten Bedienern der Vorrichtung durchführbar.

Eine vorteilhafte Weiterbildung der Erfindung sieht vor, dass die Vorrichtung eine Zuordnungseinrichtung aufweist, durch die das Sensorsignal des oder mindestens eines der Sensoren einer Extremität des Benutzers eindeutig zuordenbar ist. Eine derartige Zuordnungseinrichtung erlaubt es - beispielsweise über einen Impuls, der durch einen an einer Extremität des Benutzers angebrachten Reflektor über eine Lichtschranke ausgelöst wird - ein Sensorsignal als direkt von dieser Extremität stammend zu identifizieren. Bei bekannter Abfolge der Schritte durch die Extremitäten ist es dabei möglich, sämtliche Trittprofile den entsprechenden Extremitäten eines Benutzers zuzuordnen.

Eine weitere vorteilhafte Weiterbildung der Erfindung sieht vor, dass die Lagerung der Trittplatte mindestens ein Lager aufweist, bei dem ein erstes Lagerteil und ein zweites Lagerteil parallel geschaltet sind. Vorzugsweise weist hierbei das zweite Lagerteil eine geringe Steifigkeit und das erste Lagerteil eine variable Steifigkeit auf. Durch diese variable Steifigkeit des ersten Lagerteils ist die Gesamtsteifigkeit der Lagerung variabel.

Eine weitere vorteilhafte Weiterbildung der Erfindung sieht vor, dass das erste Lagerteil ein erstes Lagerelement hoher Steifigkeit aufweist, das mit einem zweiten Lagerelement variabler Steifigkeit in Serie geschaltet ist. Vorzugsweise ist hierbei das zweite Lagerelement des ersten Lagerteils als Luftfederung ausgebildet. Hierdurch ist es möglich, durch Variieren des Luftdrucks in der Luftfederung deren Längsausdehnung und damit den Anpressdruck des zweiten Lagerelements des ersten Lagerteils gegen der Trittplatte zu verändern. Auf diese Weise ist eine Variabilität der Steifigkeit des ersten Lagerteils und damit des gesamten Lagers gegeben.

Eine weitere vorteilhafte Weiterbildung der Erfindung sieht vor, dass die Trittplatte in Leichtbauweise ausgeführt ist. Dies ist insbesondere bei leichten Benutzern von Vorteil, da deren Eigengewicht im Verhältnis zu einer schweren Trittplatte vernachlässigbar und damit nur eine geringe Auslenkung und Beschleunigung der Trittplatte gegeben ist. Durch diese Ausbildung der Trittplatte in Leichtbauweise wird der Einfluss des Gewichts des Benutzers verstärkt und somit eine aussagekräftigere Trittanalyse ermöglicht.

Eine weitere vorteilhafte Weiterbildung der Erfindung sieht vor, dass die Trittplatte neigbar in der Vorrichtung gelagert ist. Vorzugsweise weist die Vorrichtung hierbei mindestens ein erstes Rahmenlagerelement, in dem ein die Trittplatte aufnehmender Rahmen drehbar um eine Achse quer zu einer Laufrichtung gelagert ist, und mindestens ein zweites Rahmenelement auf, dessen Länge einstellbar ist, wodurch der Abstand eines Bodenlagerpunktes und eines Rahmenlagerpunktes des zweiten Rahmenlagerelementes und dadurch die Neigung der Trittplatte veränderlich ist. Vorzugsweise ist hierbei das erste Rahmenlagerelement außerhalb einer Symmetrieachse auf einer dem zweiten Rahmenlagerelement gegenüberliegenden Seite der Symmetrieachse am Rahmen angeordnet. Durch diese Art der Lagerung und insbesondere durch die außermittige Anordnung des ersten Rahmenlagerelements wird im Gegensatz zu einer mittigen Anordnung ein Kippen um die Achse verhindert und eine stabile Lagerung der Vorrichtung sichergestellt.

Weitere vorteilhafte Weiterbildungen der Erfindung sind Gegenstand der Unteransprüche.

Weitere Merkmale und Vorteile der Erfindung sind dem nachfolgend anhand der Zeichnungen beschriebenen Ausführungsbeispiel zu entnehmen. Die Figuren zeigen im Einzelnen:
- Figur 1:: eine Seitenansicht eines Ausführungsbeispiels einer Vorrichtung zur Erfassung eines Trittprofils in einer aufwärts geneigten Position,
- Figur 2:: eine Seitenansicht des Ausführungsbeispiels der Figur 1 in einer waagrechten Position,
- Figur 3:: eine Seitenansicht des Ausführungsbeispiels der Figur 1 in einer abwärts geneigten Position.
- Figur 4:: einen Querschnitt durch das Ausführungsbeispiel der Figur 1,
- Figur 5:: eine vergrößerte Querschnittsansicht einer Trittplatte des Ausfüh- rungsbeispiels der Figur 1,
- Figur 6:: eine vergrößerte Querschnittsdarstellung eines Lagers des Ausfüh- rungsbeispiels der Figur 1,
- Figur 7:: eine alternative Ausführungsform eines Lagers des Ausführungsbei- spiels,

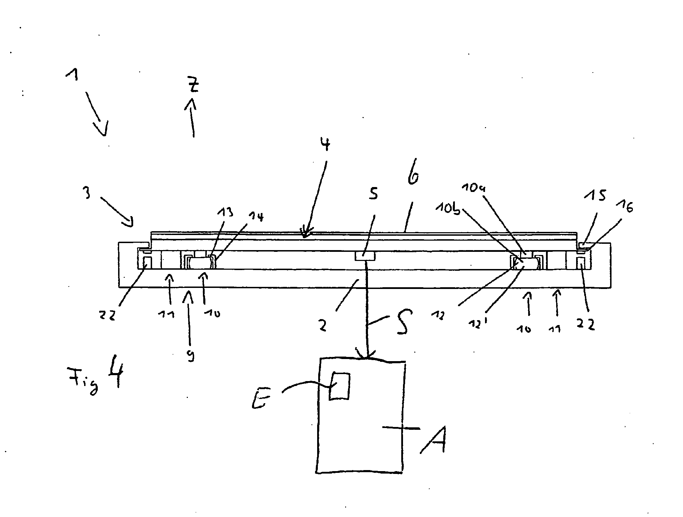

In den Figuren ist nun ein Ausführungsbeispiel einer Vorrichtung 1 zur Aufnahme eines Trittprofils eines Lebewesens, insbesondere eines Pferdes, dargestellt. Sie weist eine in einem Rahmen 2 über eine später detailliert geschilderte elastische Lagerung 3 gelagerte Trittplatte 4 auf, unterhalb der ein oder mehrere - in diesem Fall als Beschleunigungssensor ausgeführte - Sensoren 5 (siehe Figur 4) angebracht ist oder sind.

Um nun nicht - wie bei den bekannten Vorrichtungen - nur eine statische Bewegungsanalyse des Benutzers durchführen zu können, sondern es zu ermöglichen, die einzelnen Trittprofile einer dynamischen Bewegungssequenz zu analysieren , ist nun bei der beschriebenen Vorrichtung 1 vorgesehen, dass diese ein über der Trittplatte 4 angeordnetes Laufband 6 aufweist, auf dem der Benutzer diese dynamische Bewegungssequenz in einer Abfolge von in entsprechenden Trittimpulsen resultierenden einzelnen Bewegungsvorgängen - bei Pferden beispielsweise eine Abfolge von Schritt-, Trab- oder Galoppbewegungen - durchführt, wobei die einzelnen Trittimpulse des Benutzers zu entsprechenden Sensorsignales eines oder mehreren Sensoren 5 führen: Trifft nun beispielsweise eine Extremität eines Benutzers auf die Trittplatte 4, so wird diese infolge ihrer elastischen Lagerung 3 ausgelenkt und ihre Beschleunigung wird dabei durch den oder die Sensoren 5 erfasst. In einer Auswerteeinrichtung A werden die Sensorsignale S des oder der Sensoren 5 ausgewertet und vorzugsweise auf einer Anzeigeeinheit E einer Auswerteeinrichtung A dargestellt.

Der mindestens eine und vorzugsweise alle Sensoren 5 sind als ein ein- oder mehrachsiger Beschleunigungssensor ausgebildet, so dass die durch die Beaufschlagung der Trittplatte 4 durch die Extremitäten des Benutzers hervorgerufenen Beschleunigung derselben erfassbar ist. Ein derartiges Wirkprinzip des Sensors 5 ist aber nicht zwingend erforderlich. Es ist auch ein Wegmesssensor möglich, der gewährleistet, dass eine durch die Beaufschlagung der Trittplatte 4 durch die Extremität des Benutzers bewirkte Auslenkung derselben erfasst werden kann.

Wenn die Vorrichtung 1 einen einzigen Sensor 5 aufweist, ist dieser dann vorzugsweise mittig unter der Trittplatte 4 angeordnet. Wenn mehr als ein Sensor 5 vorgesehen ist, ist dann eine ortsauflösende Erfassung der die Trittplatte 4 beaufschlagenden Trittbewegungen möglich. Hierbei wird es bevorzugt, dass die Vorrichtung 1 entweder zwei oder vier oder sechs Sensoren 5 besitzt, wobei dann die zwei bzw. vier bzw. sechs Sensoren der vorderen und der hinteren Hälfte der Trittplatte 4 bzw. jeweils einem Quadranten bzw. den Enden und der Mitte der Trittplatte 4 zugeordnet sind. Hierdurch wird in einfacher Art und Weise eine separate Erfassung der durch die vorderen bzw. hinteren Extremitäten der durch den Benutzer ausgelösten Trittimpulse ermöglicht. Bei vier oder mehr Sensoren 5 ist es des weiteren möglich, jeden durch eine Extremität des vierbeinigen Benutzers ausgelösten Trittimpuls separat zu erfassen.

Da insbesondere bei Pferden je nach Gangart die Abfolge der Bewegungsschritte entweder eine Viertaktbewegung (Schritt) oder eine Zweitaktbewegung (Trab, Galopp) ist, kann den einzelnen erfassten Trittimpulsen ohne weiteres eine bestimmte Extremität des Nutzers zugeordnet werden, wenn die Zuordnung eines bestimmten Trittimpulses zu einer bestimmten Extremität bekannt ist. Diese Zuordnung kann durch einen visuellen Abgleich erfolgen. Es ist aber auch möglich, dass die Vorrichtung 1 eine nicht dargestellte Zuordnungseinrichtung - beispielsweise eine Lichtschranke - aufweist, die durch die Bewegung einer bestimmten Extremität des Benutzers - beispielsweise des linken Vorderbeines - ausgelöst wird. Das Ausgangssignal dieser Zuordnungseinrichtung wird dann der Auswerteeinrichtung A zugeführt und das zeitlich korrelierte Signal des Sensors 5 wird dann der das Ausgangssignal der Auslöseeinheit bewirkenden Extremität des Pferdes zugeordnet. Da die Abfolge der Bewegungen der Extremitäten bekannt ist, können in einfacher Art und Weise die darauffolgenden Trittimpulse den einzelnen Extremitäten zugeordnet werden.

Durch diese Maßnahmen ist einerseits die Aufnahme einer Sequenz von Trittprofilen während der zu beobachtenden Bewegungssequenz möglich, andererseits kann dieser Bewegungsablauf - während der Erfassung der Trittprofile durch die Vorrichtung 1 - gleichzeitig von einem Außenstehenden beobachtet werden, so dass die durch diese Trittprofil-Analyse gewonnenen Informationen in einfacher Art und Weise mit durch den optischen Eindruck eines Beobachters - zum Beispiel eines Arztes, Tierarztes, Therapeuten oder Trainers - gewonnen Erkenntnissen ergänzt werden können.

Die derartige gewonnene Sequenz von Trittprofilen bietet nun einem behandelnden Mediziner bzw. einem Therapeuten oder Trainer in vorteilhafter Art und Weise die Möglichkeit, nicht nur - wie bei bekannten Vorrichtungen - eine statische Belastungssituation der die Trittplatte 4 beaufschlagenden Extremitäten des Benutzers zu analysieren, sondern in vorteilhafter Weise eine zeitliche Abfolge von Bewegungsschritten - beispielsweise beim Auftreten auf die Trittplatte 4 oder während der Durchführung anderweitiger Bewegungen - zu erfassen, auszuwerten und die derart gewonnenen Informationen zur Festlegung von therapeutischen, diagnostischen, Rekonvaleszenz- oder Trainingsmaßnahmen zu verwenden.

Die beschriebene Vorrichtung 1 ermöglicht es somit in vorteilhafter Weise, dass bei Pferden in einfacher Art und Weise Lahmheitsuntersuchungen durchgeführt werden können, indem das Pferd auf das Laufband 6 gestellt und dann zur entsprechenden Bewegung - Schritt, Trab oder Galopp - angehalten wird. Die durch diese Bewegungsvorgänge ausgelösten und auf die Trittplatte 4 einwirkenden Trittimpulse werden dann vom Sensor 5 erfasst und dessen Sensorsignale S werden zu der Auswerteeinrichtung A geleitet, dort analysiert und auf der Anzeigeeinheit E der Auswerteeinrichtung A dargestellt. Durch einen Vergleich der einzelnen Trittimpulse untereinander und/oder mit vorgegebenen Trittimpulsen, die den Norm-Zustand, also zum Beispiel dem Trittimpuls eines gesunden Pferdefußes, repräsentieren, können so in einfacher Art und Weise Abweichungen festgestellt werden, die auf eine Fehlbelastung durch das Pferd, etwa bedingt durch Lahmheit, und /oder andere Irregularitäten schließen lassen.

Vorzugsweise ist es vorgesehen, dass die Auswerteeinrichtung A ein Signal erzeugt, wenn eine Abweichung eines erfassten Trittimpulses von einem anderen Trittimpuls und/oder von einem vorgegeben Norm-Trittimpuls auftritt. Eine derartige Maßnahme besitzt den Vorteil, dass hierdurch die Erkennung einer Fehlbelastung des Pferdes auch von einem ungeübten Bediener der Vorrichtung durchgeführt werden kann.

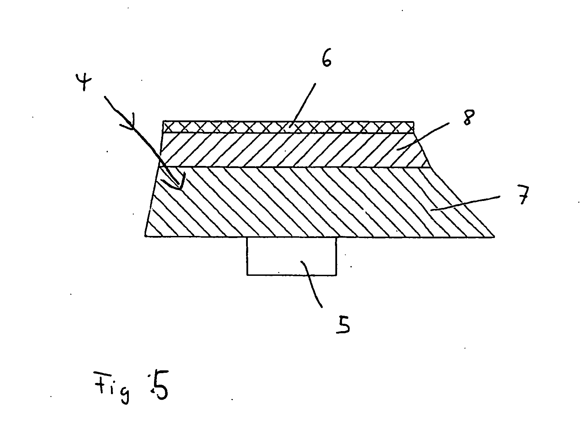

Um einen möglichst reibungsarmen Lauf des Laufbandes 6 auf der Trittplatte 4 zu ermöglichen, ist vorzugsweise vorgesehen, dass -wie aus Figur 5 ersichtlich - das Laufband 6 sich auf einer auf einer Basisplatte 7 der Trittplatte 4 angeordneten Gleitschicht 8 bewegt.

Es wird bevorzugt, dass das Laufband 6 ständig umläuft. Die hierzu erforderliche Bandrückführung ist den Figuren nicht angestellt, dem Fachmann aber bekannt.

Die Trittplatte 4 ist in einer elastischen Lagerung 3 aufgenommen. Dies besitzt den Vorteil, dass hierdurch in einfacher Art und Weise der Widerstand, welche die Trittplatte 4 den Extremitäten des Benutzers entgegensetzt, verändert und benutzerindividuell eingestellt werden kann.

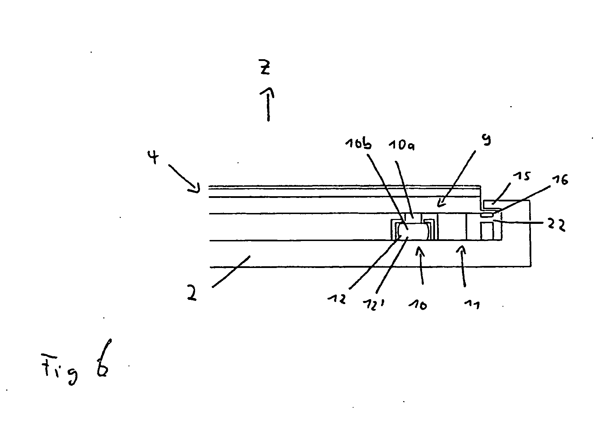

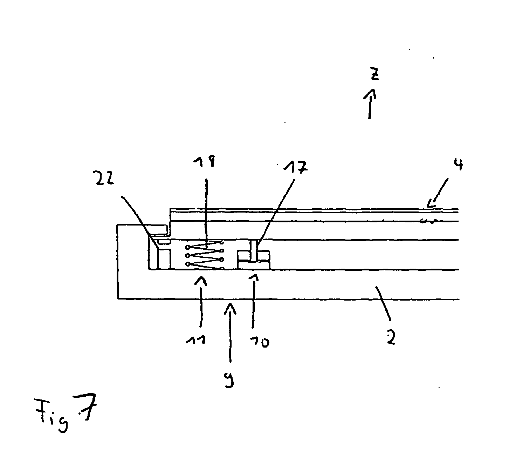

Die in den Figuren 6 und 7 im Detail dargestellte Lagerung 3 setzt sich aus mehreren, vorzugsweise vier, Lagern 9 zusammen, von denen jedes wiederum ein erstes Lagerteil 10 sowie ein zweites Lagerteil 11 aufweist, die einander parallel geschaltet sind und sowohl mit dem Rahmen 2 als auch mit der Trittplatte 4 verbunden sind. Das erste Lagerteil 10 ist dabei als Serienschaltung eines ersten Lagerelements 10a und eines zweiten Lagerelements 10b ausgeführt.

Sowohl zweites Lagerteil 11 als auch erstes Lagerelement 10a des ersten Lagerteils 10 sind als eine konstante Steifigkeit aufweisende Gummielemente ausgebildet, wobei das Gummielement des zweiten Lagerteils 11 eine geringe Steifigkeit und das des ersten Lagerelements 10a des ersten Lagerteils 10 eine hohe Steifigkeit besitzt. Als zweites Lagerelement 10b des ersten Lagerteils 10 wird hingegen eine Luftfederung 12 in Form eines Luftbalgs 12' verwendet, deren Luftmenge und dadurch Luftdruck über eine nicht dargestellte Regeleinheit regulierbar ist. Auf diese Weise besitzt das zweite Lagerteil 11 des Lagers 9 eine konstante Steifigkeit und das erste Lagerteil 10 eine variable Steifigkeit. Diese Variierbarkeit wird durch den veränderlichen Luftdruck in der Luftfederung 12 dadurch erreicht, dass sich die Luftfederung 12 bei einer Erhöhung des Luftdrucks in ihrer Länge in einer Richtung Z ausdehnt und dadurch den Anpressdruck des ersten Lagerelements 10a des ersten Lagerteils 10 gegen die Trittplatte 4 erhöht, wohingegen bei geringem Luftdruck lediglich das eine geringe Steifigkeit aufweisende zweite Lagerteil 11 wirksam ist, auf dem die Trittplatte 4 dann hauptsächlich ruht. Die Gesamtsteifigkeit des Lagers 9 ergibt sich somit aus dem Zusammenwirken von erstem Lagerteil 10 mit variabler Steifigkeit und zweitem Lagerteil 11 mit konstanter Steifigkeit.

Zur Vermeidung einer unbeschränkten unkontrollierten Bewegung der Trittplatte 4 in Z-Richtung und eines asymmetrischen Anhebens wird die Längsausdehnung der Luftfederung 12 durch eine Anschlagfläche 13 einer Halterung 14 nach oben begrenzt. Durch eine weitere Erhöhung des Luftdrucks in der Luftfederung 12 kann jedoch deren Steifigkeit über die damit einhergehende Erhöhung deren Elastizitätsmoduls weiter erhöht werden.

Durch die variable Steifigkeit der Lager 9 der Lagerung 3 ist es nunmehr möglich, die Vorrichtung 1 an das Gewicht des jeweiligen Benutzers anzupassen, indem die Steifigkeit bei einem Benutzer mit hohem Gewicht erhöht bzw. bei einem Benutzer mit niedrigerem Gewicht verringert wird. Auf diese Weise kann die Auslenkung der Trittplatte 4 eingestellt und somit der Messbereich des oder der Sensoren 5 in weitem Umfang ausgenutzt werden. Vorzugsweise verfügt die Vorrichtung 1 hierzu über in diesem Ausführungsbeispiel vier an den Ecken der Trittplatte 4 angebrachte Wegaufnehmer 22, über die die Auslenkung der Trittplatte 4 erfassbar ist, um daraufhin die Steifigkeit der Lagerung 3 einzustellen.

Durch die variabel einstellbare Steifigkeit der Lagerung 3 ist es zudem möglich, unterschiedliche Laufuntergründe zu simulieren, wobei eine hohe Steifigkeit der Lagerung 3 einen harten Untergrund und eine geringe Steifigkeit der Lagerung 3 einen weichen Untergrund simuliert.

Um eine unkontrollierte Bewegung der Trittplatte 4 in Z-Richtung wie beispielsweise beim Verlassen der Trittplatte 4 durch den Benutzer oder beim Anheben einer Extremität zu vermeiden, weist der Rahmen 2 einen Vorsprung 15 auf, gegen den ein Anschlagelement 16 der Trittplatte 4 bei einer zu großen Bewegung in Z-Richtung anschlägt und diese Bewegung somit begrenzt.

Die beschriebene Lagerung 3 erlaubt es, jedem der Lager 9 der Lagerung 3 eine unterschiedliche Steifigkeit zu verleihen und dadurch beispielsweise gezielt vordere oder hintere bzw. rechte oder linke Extremitäten des Pferdes zu trainieren, oder bei in einer Phase der Rekonvaleszenz befindlichen Pferden eine verletzte Extremität gezielt aufzubauen.

Bei dem hier geschilderten Ausführungsbeispiel entspricht die Richtung Z im wesentlichen der Wirkungslinie der Schwerkraft, wobei selbstverständlich eine Neigung der Vorrichtung 1 beispielsweise zur Simulierung einer Bergauf- bzw. einer Bergabbewegung ebenfalls denkbar ist.

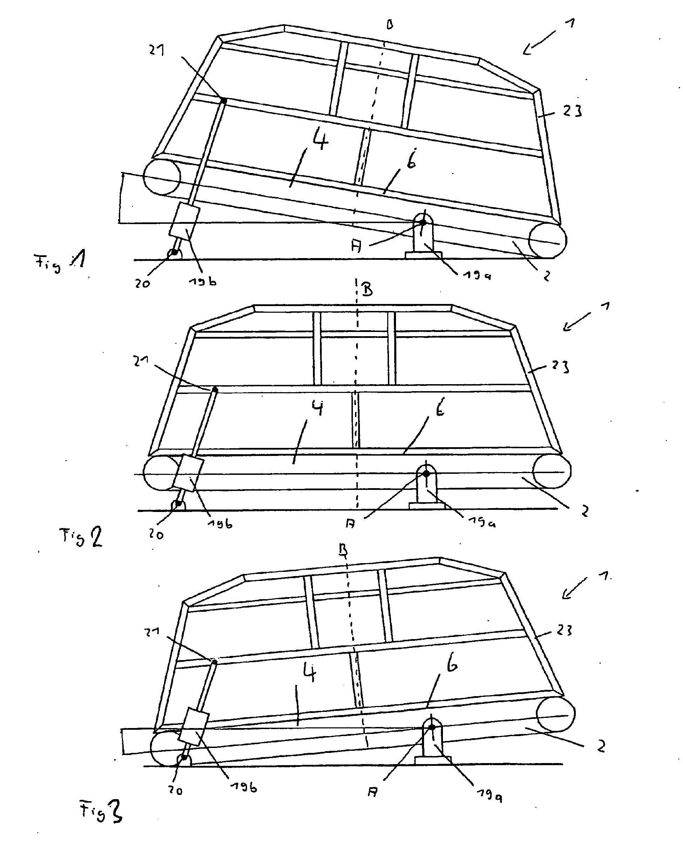

Hierzu weist die Vorrichtung 1 wie in den Figuren 1 bis 3 in einer Seitenansicht dargestellt eine Rahmenlagerung 19 auf. Sie setzt sich aus einem ersten Rahmenlagerelement 19a, in dem der Rahmen 2 drehbar um eine Achse A quer zur Laufrichtung gelagert ist und mindestens einem zweiten Rahmenlagerelement 19b zusammen, dessen Länge einstellbar ist. Das zweite Rahmenlagerelement 19b ist über einen Bodenlagerpunkt 20 mit dem Boden und über einen Rahmenlagerpunkt 21 mit einem Geländer 23 verbunden, so dass durch die variable Länge des zweiten Rahmenlagerelements 19b der Abstand des Bodenlagerpunktes 20 und des Rahmenlagerpunktes 21 veränderbar und damit die Neigung der Vorrichtung 1 einstellbar ist. Vorzugsweise ist hierbei das erste Rahmenlagerelement 19a außerhalb einer Symmetrieachse B auf der dem zweiten Rahmenlagerelement 19b gegenüberliegenden Seite der Symmetrieachse B angeordnet. Im Gegensatz zu einer mittigen Anordnung wird hierdurch eine stabilere Lage der Trittplatte 4 erreicht, da auf diese Weise ein Kippen um die Achse A verhindert bzw. erschwert wird. Vorzugsweise weist die Vorrichtung 1 hierbei zwei jeweils die Achse A einschließende erste Rahmenlagerelemente 19a und zwei in dieser Darstellung hintereinanderliegende zweite Rahmenlagerelemente 19b auf.

Wird die Vorrichtung 1 hauptsächlich von leichten Benutzern verwendet, so fällt deren Eigengewicht im Vergleich zu einer relativ schweren Trittplatte 4 kaum ins Gewicht, so dass lediglich geringe Auslenkungen der Trittplatte 4 auftreten und kein optimales Trittprofil ermittelbar ist. Aus diesem Grund wird bevorzugt, dass die Trittplatte 4 in diesem Fall in einer dem Fachmann bekannten Leichtbauweise wie zum Beispiel in Wabenkonstruktion ausgeführt ist, wodurch der Einfluss des Gewichts des Benutzers im Verhältnis zum Gewicht der Trittplatte 4 gestärkt wird und ausgeprägtere Trittprofile erhalten werden.

Alternativ zum hier geschilderten Ausführungsbeispiel der Lager 9 der Lagerung 3 sind selbstverständlich ebenfalls andere Ausführungsformen wie beispielsweise in Figur 7 dargestellt möglich, in der das erste Lagerteil 10 als regulierbarer Hydraulikdämpfer 17 und das zweite Lagerteil 11 als dazu parallel geschaltete Feder 18 ausgebildet sind. Die variable Steifigkeit des Lagers 9 wird hierbei durch die regulierbare Dämpfung des Hydraulikdämpfers 17 sichergestellt.

## Patentansprüche

1. Vorrichtung zur Aufnahme eines Trittprofils eines Lebewesens, die eine Trittplatte (4) und mindestens einen der Trittplatte (4) zugeordneten oder in ihr integrierten Sensor (5) aufweist, wobei ein Sensorsignal des mindestens einen Sensors (5) einer Auswerteeinrichtung (A) zuführbar ist, wobei die Vorrichtung (1) ein Laufband (6) aufweist, das über der Trittplatte (4) angeordnet ist, und wobei durch den mindestens einen Sensor (5) die einzelnen Trittimpulse einer aus einer Abfolge von Einzelbewegungen bestehenden dynamischen Bewegungssequenz erfassbar und die zu den einzelnen Trittimpulsen korrelierten Sensorsignale (S) der Auswerteeinrichtung (A) zuführbar sind, **dadurch gekennzeichnet, dass** die Trittplatte (4) mittels einer Lagerung (3) elastisch in der Vorrichtung (1) gelagert ist, dass der oder mindestens einer der Sensoren (5) als ein eine Beschleunigung und/oder eine Auslenkung der Trittplatte (4) erfassender Sensor (5) ausgebildet ist.

2. Vorrichtung nach Anspruch 1, **dadurch gekennzeichnet, dass** die Vorrichtung einen die gesamte Trittplatte (4) erfassenden Sensor (5) oder zwei Sensoren (5), die jeweils einer Hälfte der Trittplatte (4) zugeordnet sind, oder vier Sensoren (5), die jeweils einem Quadranten der Trittplatte (4) zugeordnet sind, aufweist.

3. Vorrichtung nach einem der vorangehenden Ansprüche, **dadurch gekennzeichnet, dass** die zu den einzelnen Trittimpulsen der dynamischen Bewegungssequenz korrelierten Sensorsignale (S) auf einer Anzeigeeinheit (E) der Auswerteeinrichtung (A) darstellbar sind, und dass durch die Auswerteeinrichtung (A) die einzelnen Trittimpulse der dynamischen Bewegungssequenz miteinander und/oder mit vorgegebenen Norm-Trittimpulsen vergleichbar sind.

4. Vorrichtung nach Anspruch 3, **dadurch gekennzeichnet, dass** durch die Auswerteeinrichtung bei einer Abweichung eines Trittimpulses von einem anderen Trittimpuls und/oder von einem vorgegebenen Norm-Trittimpuls ein Signal erzeugbar ist.

5. Vorrichtung nach einem der vorangehenden Ansprüche, **dadurch gekennzeichnet, dass** die Vorrichtung (1) eine Zuordnungseinrichtung aufweist, durch die ein Sensorsignal (S) des oder mindestens eines der Sensoren (5) einer bestimmten Extremität eines Benutzers der Vorrichtung (1) eindeutig zuordenbar ist.

6. Vorrichtung nach Anspruch 1, **dadurch gekennzeichnet, dass** die Steifigkeit der Lagerung (3) der Trittplatte (4) einstellbar ist

7. Vorrichtung nach einem der Ansprüche 1 oder 6, **dadurch gekennzeichnet, dass** die Lagerung (3) der Trittplatte (4) mindestens ein Lager (9) aufweist, bei dem ein erstes Lagerteil (10) und ein zweites Lagerteil (11) parallel geschaltet sind, wobei vorzugsweise das zweite Lagerteil (11) eine geringe Steifigkeit und das erste Lagerteil (10) eine variable Steifigkeit aufweist.

8. Vorrichtung nach Anspruch 7, **dadurch gekennzeichnet, dass** das erste Lagerteil (10) ein erstes Lagerelement (10a) hoher Steifigkeit aufweist, das mit einem zweiten Lagerelement (10b) variabler Steifigkeit In Serie geschaltet ist.

9. Vorrichtung nach einem der Ansprüche 7 oder 8, **dadurch gekennzeichnet, dass** das zweite Lagerelement (10b) des ersten Lagerteils (10) als pneumatische, hydraulische, mechanische oder elektromagnetische Federung oder als eine Luftfederung (12) ausgebildet ist

10. Vorrichtung nach Anspruch 9, **dadurch gekennzeichnet, dass** die Federung (12) eine Begrenzung für eine Längsausdehnung in der vertikalen Richtung (Z) aufweist.

11. Vorrichtung nach einem der Ansprüche 7 bis 9, **dadurch gekennzeichnet, dass** das erste Lagerteil (10) ein regelbarer Hydraulikdämpfer (17) und das zweite Lagerteil (11) eine Feder (18) ist.

12. Vorrichtung nach einem der vorangehenden Ansprüche, **dadurch gekennzeichnet, dass** die Trittplatte (4) eine Begrenzung der Beweglichkeit in einer vertikalen Richtung (Z) aufweist.

13. Vorrichtung nach einem der vorangehenden Ansprüche, **dadurch gekennzeichnet, dass** ein Rahmen (2) neigbar in der Vorrichtung (1) gelagert ist. insbesondere dass die Vorrichtung (1) eine Rahmenlagerung (19) mit mindestens einem ersten Rahmenlagerelement (19a), in dem der Rahmen (2) drehbar um eine Achse (A) quer zu einer Laufrichtung gelagert ist, und mindestens einem zweiten Rahmenlagerelement (19b) aufweist, dessen Länge einstellbar ist, wodurch der Abstand eines Bodenlagerpunktes (20) und eines Rahmenlagerpunktes (21) des zweiten Rahmenlagerelementes (19b) und **dadurch** die Neigung der Trittplatte (4) veränderlich ist, wobei insbesondere das erste Rahmenlagerelement (19a) außerhalb einer Symmetrieachse (B) auf einer dem zweiten Rahmenlagerelement (19b) gegenüberliegenden Seite der Symmetrieachse (B) am Rahmen (2) angeordnet ist.

14. Verfahren zur Aufnahme eines Trittprofils eines Lebewesens, insbesondere eines Pferdes, mit dem eine Beaufschlagung einer Trittplatte (4) einer Vorrichtung von mindestens einem Sensor (5) erfasst wird und das aus der Beaufschlagung der Trittplatte (4) resultierende Sensorsignal (S) einer Auswerteeinrichtung (A) zugeführt wird, wobei dass zur Erfassung einzelner Trittimpulse einer aus einer Abfolge von Trittimpulsen bestehenden dynamischen Bewegungssequenz das Lebewesen auf einem über der Tragplatte (4) angeordneten Laufband (6) angeordnet wird, und wobei durch den mindestens einen Sensor (5) die einzelnen Trittimpulse einer aus einer Abfolge von Einzelbewegungen bestehenden dynamischen Bewegungssequenz erfasst und die zu den einzelnen Trittimpulsen korrelierten Sensorsignale (S) der Auswerteeinrichtung (A) zu geführt werden, **dadurch gekennzeichnet, dass** die Trittplatte (4) elastisch in der Vorrichtung (1) gelagert wird, und dass als der Sensor (5) oder als mindestens einer der Sensoren (5) ein Beschleunigungssensor oder ein Wegmesssensor verwendet wird, wodurch die durch die Beaufschlagung der Trittplatte (4) durch die Extremitäten des Benutzers hervorgerufene Beschleunigung und/oder Auslenkung derselben (4) erfasst wird.

## Claims

1. Device for capturing a gait pattern of a living being, comprising a tread plate (4) and at least one sensor (5), associated with the tread plate (4) or integrated into it, a sensor signal of the at least one sensor (5) being supplyable to an evaluation device (A), wherein the device (1) comprises a treadmill (6) which is superposed to the tread plate (4), and wherein the at least one sensor (5) allows the individual tread pulses of a dynamic sequence of movements, consisting of a sequence of individual movements, to be detected and the sensor signals (S) correlated with the individual tread pulses to be supplied to the evaluation device (A), **characterized in that** the tread plate (4) is elastically supported in the device (1) by a suspension (3) and that the sensor or at least one of the sensors (5) is a sensor that captures an acceleration and/or displacement of the tread plate (4).

2. The device as defined in Claim 1, **characterized in that** the device comprises a sensor (5) that covers the whole tread plate (4), or comprises two sensors (5), each assigned to one half of the tread plate (4), or comprises four sensors (5), each assigned to one quadrant of the tread plate (4).

3. The device as defined in any of the preceding claims, **characterized in that** the sensor signals (5) correlated with the individual tread pulses of the dynamic sequence of movements can be displayed on a display unit (E) of the evaluation device (A), and that the individual tread pulses of the dynamic sequence of movements can be compared one with the other and/or with predefined standard tread pulses by the evaluation device (A).

4. The device as defined in Claim 3, **characterized in that** a signal can be produced by the evaluation device when a tread pulse deviates from another tread pulse and/or from a predefined standard tread pulse.

5. The device as defined in any of the preceding claims, **characterized in that** the device (1) comprises a correlation device by which a sensor signal (S) of the sensor or the at least one of the sensors (5) can be clearly correlated with a given extremity of a user of the device (1).

6. The device as defined in Claim 1, **characterized in that** the stiffness of the suspension (3) of the tread plate (4) can be adjusted.

7. The device as defined in Claim 1 or Claim 6, **characterized in that** the suspension (3) of the tread plate (4) comprises at least one bearing (9) in which a first bearing part (10) and a second bearing part (11) are arranged in parallel, and that preferably the second bearing part (11) has a low stiffness and the first bearing part (10) has a variable stiffness.

8. The device as defined in Claim 7, **characterized in that** the first bearing part (10) comprises a first bearing element (10a) of high stiffness which is arranged in series with a second bearing element (10b) of variable stiffness.

9. The device as defined in any of Claims 7 or 8, **characterized in that** the second bearing element (10b) of the first bearing part (10) is configured as a pneumatic, hydraulic, mechanical or electromagnetic suspension or as an air suspension (12).

10. The device as defined in Claim 9, **characterized in that** the suspension (12) is provided with a limiter for the extension in length in the vertical direction (Z).

11. The device as defined in any of Claims 7 to 9, **characterized in that** the first bearing part (10) is an adjustable hydraulic damper (17) and the second bearing part (11) is a spring (18).

12. The device as defined in any of the preceding claims, **characterized in that** the tread plate (4) comprises a limiter for the mobility in a vertical direction (Z).

13. The device as defined in any of the preceding claims, **characterized in that** a frame (2) is supported tiltably in the device (1), in particular that the device (1) comprises a frame support (19) with at least one frame support element (19a) in which the frame (2) is provided for rotation about an axis (A), transversely to a direction of motion, and at least one second frame support element (19b), which can be adjusted in length for varying the spacing between a ground bearing point (20) and a frame bearing point (21) of the second frame support element (19b) and, accordingly, the inclination of the tread plate (4), and that in particular the first frame support element (19a) is arranged on the frame (2) in a position offset from an axis of symmetry (B), on the side of the axis of symmetry (B) opposite the second frame support element (19b).

14. Method for capturing the gait pattern of a living being, especially of a horse, wherein an impact of a tread plate (49) of a device is captured by at least one sensor (5), and the sensor signal (S) resulting from said impact to the tread plate (4) is supplied to an evaluation device (A), wherein the living being, for capturing individual tread pulses out of a dynamic sequence of movements that consists of a sequence of tread pulses, is placed on a treadmill (6) which is superposed to the tread plate (4), and wherein the individual tread pulses of a dynamic sequence of movements, consisting of a sequence of individual movements, are captured by the at least one sensor (5) and the sensor signals (S) correlated with the individual tread pulses are supplied to the evaluation device (A), **characterized in that** the tread plate (4) is elastically supported in the device (1) and that the sensor (5), or at least one of the sensors (5), is an acceleration sensor or a displacement sensor that captures an acceleration and/or a displacement of the tread plate (4) produced by the extremities of the user acting on the tread plate (4).

## Revendications

1. Dispositif destiné à enregistrer un profil de démarche d'un être vivant, lequel dispositif comporte un plateau de marche (4) et au moins un capteur (5), associé au plateau de marche (4) ou intégré dans celui-ci, un signal délivré par ledit au moins un capteur (5) pouvant être acheminé vers un dispositif d'analyse (A), ledit dispositif (1) comportant un tablier roulant (6), qui est disposé au-dessus du plateau de marche (4), et les différentes impulsions de marche d'une séquence dynamique de mouvements, formée par une succession de mouvements individuels, pouvant être détectées par ledit au moins un capteur (5), et les signaux (S) du capteur, corrélés avec les différentes impulsions de marche, pouvant être acheminés vers le dispositif d'analyse (A), **caractérisé en ce que** le plateau de marche (4) est monté de manière élastique dans le dispositif (1) au moyen d'un système de montage (3), **en ce que** le ou au moins un des capteurs (5) est réalisé sous la forme d'un capteur (5) détectant une accélération et/ou une déviation du plateau de marche (4).

2. Dispositif selon la revendication 1, **caractérisé en ce que** ledit dispositif comporte un capteur (5) détectant la totalité du plateau de marche (4), ou deux capteurs (5), qui sont associés chacun à une moitié du plateau de marche (4), ou quatre capteurs (5) qui sont associés chacun à un quadrant du plateau de marche (4).

3. Dispositif selon l'une des revendications précédentes, **caractérisé en ce que** les signaux (S) du capteur, corrélés avec les différentes impulsions de marche de la séquence dynamique de mouvements, peuvent être représentés sur une unité d'affichage (E) du dispositif d'analyse (A), et **en ce que** les différentes impulsions de marche de la séquence dynamique de mouvements peuvent être comparées, par le dispositif d'analyse (A), les unes aux autres et/ou avec des impulsions de marche standard prédéfinies.

4. Dispositif selon la revendication 3, **caractérisé en ce que**, en cas de divergence d'une impulsion de marche par rapport à une autre impulsion de marche et/ou par rapport à une impulsion de marche standard prédéfinie, un signal peut être délivré par le dispositif d'analyse.

5. Dispositif selon l'une quelconque des revendications précédentes, **caractérisé en ce que** ledit dispositif (1) comporte une unité d'affectation, par laquelle un signal (S) du ou d'au moins un des capteurs (5) peut être associé sans équivoque à une extrémité de membre déterminée d'un utilisateur du dispositif (1).

6. Dispositif selon la revendication 1, **caractérisé en ce que** la rigidité du système de montage (3) du plateau de marche (4) est réglable.

7. Dispositif selon la revendication 1 ou 6, **caractérisé en ce que** le système de montage (3) du plateau de marche (4) comporte au moins un palier (9), dans lequel une première partie (10) et une deuxième partie (11) sont montées en parallèle, sachant que, de préférence, la deuxième partie de palier (11) possède une faible rigidité et la première partie de palier (10) possède une rigidité variable.

8. Dispositif selon la revendication 7, **caractérisé en ce que** la première partie de palier (10) comporte un premier élément de palier (10a) avec une rigidité élevée, lequel est monté en série avec un deuxième élément de palier (10b) à rigidité variable.

9. Dispositif selon la revendication 7 ou 8, **caractérisé en ce que** le deuxième élément de palier (10b) de la première partie de palier (10) est réalisé sous la forme d'une suspension pneumatique, hydraulique, mécanique ou électromagnétique ou sous la forme d'une suspension à air (12).

10. Dispositif selon la revendication 9, **caractérisé en ce que** la suspension (12) comporte une limite pour une extension en longueur dans la direction verticale (Z).

11. Dispositif selon l'une quelconque des revendications 7 à 9, **caractérisé en ce que** la première partie de palier (10) comporte un amortisseur hydraulique (17) réglable et la deuxième partie de palier (11) comporte un ressort (18).

12. Dispositif selon l'une quelconque des revendications précédentes, **caractérisé en ce que** le plateau de marche (4) comporte une limite de la mobilité dans une direction verticale (Z).

13. Dispositif selon l'une quelconque des revendications précédentes, **caractérisé en ce que** le bâti (2) est disposé de manière inclinable dans ledit dispositif (1), en particulier en ce que ledit dispositif (1) comporte un système de montage du bâti (19) avec au moins un premier élément d'appui (19a), dans lequel le bâti (2) est monté rotatif autour d'un axe (A) transversalement à une direction de déplacement, et au moins un deuxième élément d'appui (19b), dont la longueur est réglable, moyennant quoi il est possible de modifier la distance entre un point d'appui sur le sol (20) et un point d'appui sur le bâti (21) du deuxième élément d'appui (19b) et, de ce fait, l'inclinaison du plateau de marche (4), sachant qu'en particulier le premier élément d'appui (19a) est disposé en dehors d'un axe de symétrie (B) sur un côté de l'axe de symétrie (B), opposé au deuxième élément d'appui (19b).

14. Procédé destiné à enregistrer un profil de démarche d'un être vivant, en particulier un cheval, par lequel procédé une sollicitation d'un plateau de marche (4) d'un dispositif est détectée par au moins un capteur (5) et le signal (S) du capteur, résultant de la sollicitation du plateau de marche (4), est acheminé vers un dispositif d'analyse (A), sachant que pour détecter des impulsions de marche individuelles d'une séquence dynamique de mouvements, formée par une succession d'impulsions de marche" l'être vivant est posé sur un tablier roulant (6), agencé au-dessus du plateau de marche (4), et sachant que les différentes impulsions de marche d'une séquence dynamique de mouvements, formée par une succession de mouvements individuels, sont détectées par ledit au moins un capteur (5), et les signaux (S) du capteur, corrélés avec les différentes impulsions de marche, sont acheminés vers le dispositif d'analyse (A), **caractérisé en ce que** le plateau de marche (4) est monté de manière élastique dans le dispositif (1), **en ce que** le ou au moins un des capteurs (5) utilisé est un capteur d'accélération ou un capteur de mesure du déplacement, par lequel est enregistrée l'accélération, générée par la sollicitation du plateau de marche (4) par les extrémités de membre de l'utilisateur et/ou la déviation dudit plateau de marche (4).
